# EUROPEAN PATENT SPECIFICATION

(11) **EP 4 194 081 B1**
(45) Date of publication and mention of the grant of the patent: **26.03.2025**
(21) Application number: 22725346.5
(22) Date of filing: 18.02.2022
(51) Int. Cl.: B01J 6/00, C07C 17/25, F27B 1/22, F27B 1/24, F27B 1/26, B01J 19/00, B01J 19/24

(54) **NOVEL ANTI-COKING VDF CRACKING FURNACE SYSTEM AND METHOD FOR USING SAME**
NEUARTIGES OFENSYSTEM UND VERFAHREN FÜR VERKOKUNGSSCHUTZ-VDF-CRACKEN
NOUVEAU SYSTÈME DE FOUR DE CRAQUAGE VDF ANTI-COKÉFACTION ET SON PROCÉDÉ D'UTILISATION

(30) Priority: 22.10.2021 CN 202111231012
(43) Date of publication of application: 14.06.2023
(73) Proprietor: Jiangsu Gelan Environmental Technology Co., Ltd., Taizhou, Jiangsu 214500 (CN)
(72) Inventor: JIANG, Wenchun, Taizhou, Jiangsu 214500 (CN); ZHANG, Weihong, Taizhou, Jiangsu 214500 (CN); ZHANG, Chunxue, Taizhou, Jiangsu 214500 (CN); YUAN, Buhua, Taizhou, Jiangsu 214500 (CN)
(74) Representative: Bandpay & Greuter
(86) International application number: PCT/CN2022/076739
(87) International publication number: WO 2023/065575

(56) References cited:
- EP-A2- 0 276 775
- CN-A- 107 543 413
- CN-A- 107 543 413
- CN-A- 113 976 046
- CN-U- 203 209 060
- CN-U- 205 603 486
- CN-U- 205 603 486
- CN-U- 207 456 236
- CN-U- 210 545 014
- DE-A1- 2 021 559
- US-A- 5 728 906
- US-A1- 2009 252 660

## Description

### TECHNICAL FIELD

The present invention relates to the field of cracking furnace technologies, in particular to a novel anti-coking VDF cracking furnace system and a use method thereof.

### BACKGROUND

Vinylidene fluoride (VDF, CH₂CF₂) is one of important monomers in the fluorine chemical industry, which is mainly used to produce polyvinylidene fluoride and fluororubber. There are many preparation methods for preparing vinylidene fluoride. At present, vinylidene fluoride is mainly prepared through high temperature cracking with 1-chloro-1,1-difluoroethane (HCFC-142b or R142a) as raw material. Cracking reaction usually proceeds in a cracking furnace. A cracking furnace including a pre-heating step of the fluid to be processed, which then flows downwards in spiral tube inside the furnace and then is further led to a quencher is known from EP 0 276 775-A2. Because the cracking reaction is sensitive to temperature, a conversion rate is relatively low in a case of too low temperature, and coking is easy to occur in a case of too high temperature. Currently, in the VDF production industry, the furnace tubes of the cracking furnaces are mostly in the form of U-shape tandem structure and the structure is easy to coke at a bottom bent tube part, which may reduce the yield rate of the cracking furnace and shorten its operation period. Furthermore, the coking fouling on the furnace tube reduces heat transfer coefficient, increases the temperature of the furnace wall, leading to larger energy consumption. In addition, the deposition of the coking fouling reduces an inner diameter of the furnace tube, increases pressure, lowers production, increases operation costs and shortens the service life of the furnace tube. In a case of severe coking, the tube may be clogged and the furnace has to be stopped for decoking, which has direct impact on normal production. Further, a product obtained through cracking reaction in the cracking furnace has high temperature and thus usually needs to be cooled by a heat exchanger for heat exchange and temperature reduction. Carbon powder in the cracking product may settle down and coke up, which not only affects conversion efficiency but also requires regular shutdown for decoking, thus affecting normal production and lowering production efficiency.

### SUMMARY

In order to solve the above technical problems, the present invention provides a novel anti-coking VDF cracking furnace system, which can reduce not only coking in a furnace tube of a cracking furnace but also coking in a tube of a heat exchanger, thereby extending a cleaning period of the furnace tube of the cracking furnace and the tube of the heat exchanger.

In order to achieve the above purpose, the present invention adopts the following technical solution.

There is provided a novel anti-coking VDF cracking furnace system as defined in claim 1, including: a cracking furnace, a waste heat recoverer and quencher, where the cracking furnace includes a cracking furnace body, a spiral furnace tube and an up-down zoning heating device are disposed in the cracking furnace body, and a cracking furnace feed opening and a cracking furnace discharge opening are disposed respectively at the top and bottom of the spiral furnace tube.

The waste heat recoverer includes a first shell-and-tube heat exchanger and a first horizontal tank located at the bottom of the first shell-and-tube heat exchanger. A first j acket is disposed on the outside of the first horizontal tank. The cracking furnace discharge opening is in communication with a feed opening of the first horizontal tank of the waste heat recoverer through a pipe line. A discharge opening of the first horizontal tank is in communication with a tube pass feed opening of the first shell-and-tube heat exchanger. A cold material is introduced into a shell pass of the first shell-and-tube heat exchanger. A shell pass discharge opening of the first shell-and-tube heat exchanger is in communication with a feed opening of the first jacket, and a discharge opening of the first jacket is in communication with the cracking furnace feed opening.

The quencher includes two paralleled second shell-and-tube heat exchangers and a second horizontal tank is disposed at the bottom of the two second shell-and-tube heat exchangers. A tube pass discharge opening of the first shell-and-tube heat exchanger is in communication with a tube pass feed opening of one second shell-and-tube heat exchanger of the quencher through a pipe line. Tube pass bottoms of the two second shell-and-tube heat exchangers are both in direct communication with the second horizontal tank. A tube pass discharge opening of the other second shell-and-tube heat exchanger of the quencher is connected through a pipe line to a product collection procedure, and cooling water is introduced into shell passes of the two second shell-and-tube heat exchangers.

In the above technical solution, the furnace tube is designed as spiral furnace tube such that the material is enabled to flow insides at a changing flow rate and be in a continuous descending state, thus avoiding coking due to too high temperature resulting from deposition of the material at a local part of the furnace tube, and further, there is almost no laminar flow of tube wall, the material is uniformly heated and side reaction is reduced. In addition, by use of the waste heat recovery device, the waste heat of the reaction product can be fully utilized to preheat the cold material, such that it can reach a given temperature before entering the cracking furnace, thereby reducing the energy consumption of the cracking furnace. By disposing the quencher, the reaction product can be further cooled to 50 to 60°C with a circulating cooling water as cooling medium.

Furthermore, the zoning heating device includes at least two electric heating zones arranged up and down. Each electric heating zone is provided with an independent electric heating system, the electric heating system is disposed at an inner wall of the cracking furnace body, and each electric heating zone is provided with a furnace wall temperature measuring point and a material temperature measuring point. The furnace wall temperature measuring point is disposed at the inner wall of the cracking furnace body, and the material temperature measuring point is disposed at an outer wall of the spiral furnace tube. Each of the furnace wall temperature measuring point, the material temperature measuring point and the electric heating system is connected with a temperature control system.

The above zoning heating device includes at least two electric heating zones, each of which is independently adjustable in heating power. Each electric heating zone is provided with the furnace wall temperature measuring point and the material temperature measuring point such that the heating power of the electric heating system of each electric heating zone can be accurately controlled based on measured material temperature and furnace wall temperature. Thus, the material temperature of each electric heating zone can be controlled so as to keep a reaction temperature in the furnace body constant in a case of continuous change of the flow rate of the material. By keeping the reaction temperature constant, the conversion rate of the cracking reaction can be increased, coking is prevented and the energy consumption of the cracking furnace can be reduced. Furthermore, in order to ensure the accuracy and stability of the zoning heating device, the temperature control system adopts an MPID automatic temperature control system and a heating voltage of the electric heating system is 380/220V.

Furthermore, the spiral furnace tube spirals down axially along the cracking furnace body and is close to the inner wall of the cracking furnace body, and a spacing of adj acent spiral furnace tubes is 2 to 4 times a diameter of the spiral furnace tube.

In the above technical solution, the furnace tube is designed as spiral and made close to the inner wall of the cracking furnace body, and the spacing of the furnace tube can be adjusted, such that an inner side of the furnace tube may also be subjected to heat radiation, thereby ensuring a constant temperature in the spiral furnace tube. Further, designed as an up-down spiral structure, the furnace tube may also be better combined with the zoning heating device to help control the temperature of each zone and keep the temperature constant.

Furthermore, an inner protruding tube is disposed in the first horizontal tank of the waste heat recoverer and the cracking furnace discharge opening is connected with the inner protruding tube through a pipe line.

A flow guide plate is disposed at a middle upper part of the first horizontal tank, and the flow guide plate is disposed at a side close to an outlet end of the inner protruding tube.

In the above technical solution, the carbon powder in the reaction product can be precipitated and intercepted in the first horizontal tank and thus prevented from entering the shell-and-tube heat exchanger for settling and coking. In this case, the heat exchange efficiency can be increased and it is not required to perform regular shutdown for decoking, ensuring normal production.

Furthermore, a second jacket is disposed at the outside of the second horizontal tank, and cooling water is introduced into the second jacket.

Furthermore, the cracking furnace body is a closed furnace body in which nitrogen is filled to maintain a micro-positive pressure, and an explosion opening is disposed at the top of the cracking furnace body. The explosion opening is connected with an external waste gas treatment system through a pipe line. The explosion opening is provided with an explosion disk.

In the above technical solution, if the spiral furnace tube explodes, the explosion disk will explode and a poisonous gas may enter the waste gas treatment system from the explosion opening through a pipeline, avoiding leakage of the poisonous gas.

The present invention further provides a use method of the novel anti-coking VDF cracking furnace system as defined in claim 1, including the following steps:
(1) the furnace wall temperature measuring point and the material temperature measuring point of each electric heating zone are connected to the temperature control system in advance, and the electric heating system of the each electric heating zone is connected to the temperature control system, and then a heating temperature range of each electric heating zone and temperature ranges of the furnace wall temperature measuring point and the material temperature measuring point of each electric heating zone are set, wherein, when the temperature control system detects the temperatures of the furnace wall temperature measuring point and the material temperature measuring point are not within the corresponding set ranges, the temperature control system sends signals to automatically adjust a heating power of the electric heating system, so as to keep a material reaction temperature constant in the cracking furnace body;
(2) after setup is completed according to step (1), a cold material is introduced into the shell pass of the first shell-and-tube heat exchanger of the waste heat recoverer, and passed into the first jacket through the shell pass discharge opening of the first shell-and-tube heat exchanger and then introduced into the cracking furnace feed opening through the discharge opening of the first j acket to perform reaction in the cracking furnace body, and then a reaction product enters the first horizontal tank and the tube pass of the first shell-and-tube heat exchanger sequentially from the cracking furnace discharge opening so as to perform heat exchange for temperature reduction while the cold material is heated for heat absorption;
(3) the reaction product flows out of the tube pass of the first shell-and-tube heat exchanger and enters the tube pass of one second shell-and-tube heat exchanger, the second horizontal tank and the tube pass of the other second shell-and-tube heat exchanger of the quencher sequentially to perform heat exchange for temperature reduction, and then is discharged from the top tube pass discharge opening of the second shell-and-tube heat exchanger to the product collection procedure.

Furthermore, the heat exchange process in step (2) is as follows: the reaction product enters the first horizontal tank through the inner protruding tube from the cracking furnace discharge opening to perform heat exchange with the cold material in the first jacket so as to preheat the cold material of the first jacket while a carbon powder in the reaction product is precipitated in the first horizontal tank; and then, the reaction product enters the tube pass of the first shell-and-tube heat exchanger through the top discharge opening of the first horizontal tank to perform heat exchange with the cold material in the shell pass of the first shell-and-tube heat exchanger so as to preheat the cold material in the shell pass of the first shell-and-tube heat exchanger, that is, the cold material entering from the shell pass of the first shell-and-tube heat exchanger is preheated two times and then conveyed to the cracking furnace feed opening through the discharge opening of the first jacket.

The heat exchange process in step (3) is as follows: the reaction product enters the tube pass of one second shell-and-tube heat exchanger of the quencher from the tube pass of the first shell-and-tube heat exchanger to perform a first heat exchange and then enters the second horizontal tank at the bottom of the second shell-and-tube heat exchanger to perform a second heat exchange in the second horizontal tank while the second horizontal tank intercepts the carbon powder in the reaction product, and then enters the tube pass of the other second shell-and-tube heat exchanger of the quencher for a third heat exchange and then is discharged from the tube pass discharge opening of the second shell-and-tube heat exchanger to the product collection procedure.

The present invention has the following beneficial effects.
(1) In the present invention, the furnace tube is designed as spiral furnace tube such that the material is enabled to flow insides at a changing flow rate and be in a continuous descending state, thus avoiding coking due to too high temperature resulting from deposition of the material at a local part of the furnace tube, and further, there is almost no laminar flow of tube wall, the material is uniformly heated and side reaction is reduced. For example, the content of fluorine ions in hydrochloric acid is stable between 400 ppm and 500ppm, and for an ordinary straight tube cracking furnace, the content of fluorine ions in hydrochloric acid is about 1500 ppm.
(2) By use of the waste heat recovery device, the waste heat of the reaction product can be fully utilized to preheat the cold material, such that it can reach a given temperature before entering the cracking furnace. Further, the waste heat recovery rate of the present invention is high. After waste heat recovery, the reaction product can be cooled to 300°C and the cold material can be preheated to 300°C, thereby reducing the energy consumption of the cracking furnace. By disposing the quencher, the reaction product can be further cooled to 50 to 60°C with a circulating cooling water as cooling medium.
(3) The zoning heating device is disposed and the furnace tube is designed as spiral. By cooperation of the zoning heating device and the spiral furnace tube, the temperature of each electric heating zone can be accurately controlled to keep the material reaction temperature of each zone constant in a case of continuous change of flow rate of the material. In this way, the conversion rate of the cracking reaction is increased, coking resulting from a too high temperature of a zone is avoided, and the energy consumption of the cracking furnace can be reduced.
(4) The temperature control system of the present invention adopts an MPID automatic temperature control system and a heating voltage of the electric heating system is 380/220V. There is no harmonic wave pollution of power grid, the temperature can be controlled to be within ±1°C. Along with change of material, the power is automatically compensated to achieve fully automatic control with small power consumption and high heating efficiency. In the effective heating zone, the temperature is constant and no local overheat can be generated. Compared with the original U-shaped vertical tube cracking furnace using low voltage and large current, the power consumption per ton can be lowered to 500kWh, saving more than 30%.
(5) By disposing the first horizontal tank and the flow guide plate, a small amount of carbon powder generated by reaction can be intercepted to further reduce coking in the shell-and-tube heat exchanger and extend the cleaning period. In a case of full load operation, no decoking is required in six months or even one year or more of continuous operation.
(6) The cracking furnace body is a closed furnace body on the top of which an explosion disk is disposed. Compared with the original vertical tube cracking furnace of opening structure, if the furnace tube explodes, the explosion disk will explode, and a poisonous gas may enter the waste gas treatment system from the explosion opening through a pipeline, avoiding leakage of the poisonous gas.

### BRIEF DESCRIPTIONS OF THE DRAWINGS

In order to clearly describe the technical solutions in the embodiments of the present invention or the prior art, the drawings required for descriptions of the embodiments or prior arts will be briefly introduced. Apparently, the drawings in the following descriptions are some embodiments of the present invention, and other drawings may be obtained by those skilled in the art based on these drawings without making creative work.
FIG. 1 is a schematic diagram illustrating an entire structure of an embodiment of the present invention.
FIG. 2 is a structural schematic diagram illustrating a cracking furnace.

Numerals of the drawings are described below: 1. cracking furnace, 101. cracking furnace body, 102. spiral furnace tube, 103. cracking furnace feed opening, 104. cracking furnace discharge opening, 105. furnace lining, 106. explosion opening, 2. waste heat recoverer, 201. first shell-and-tube heat exchanger, 202. first horizontal tank, 203. first jacket, 204. inner protruding tube, 205. flow guide plate, 3. quencher, 301. second shell-and-tube heat exchanger, 302. second horizontal tank, 303. second jacket, 4. zoning heating device, 401. first electric heating zone, 402. second electric heating zone, 403. third electric heating zone, 404. fourth electric heating zone, 405. furnace wall temperature measuring point, and 406. material temperature measuring point.

### DETAILED DESCRIPTIONS OF EMBODIMENTS

The present invention provides a novel anti-coking VDF cracking furnace system and a use method thereof. In order to make the object, technical solutions and effects of the present invention clearer, the present invention will be further described in details. It should be understood that the specific embodiments described herein are used only to explain the present invention rather than limit the present invention.

In the descriptions of the present invention, it is to be understood that, the orientations and positional relationships indicated by the terms "top", "bottom", "front", "rear", "upper", "lower", "inner" and "outer" and the like are based on the orientations or positional relationships shown in the drawings and used only to facilitate descriptions of the present invention and simplified descriptions rather than indicate or imply the indicated apparatus or element must have a specific orientation, and be constructed and operated at a specific orientation. Thus, the orientations or positional relationships shall not be understood as limiting of the present invention.

The present invention will be described below in details in combination with specific embodiments.

### Embodiment 1

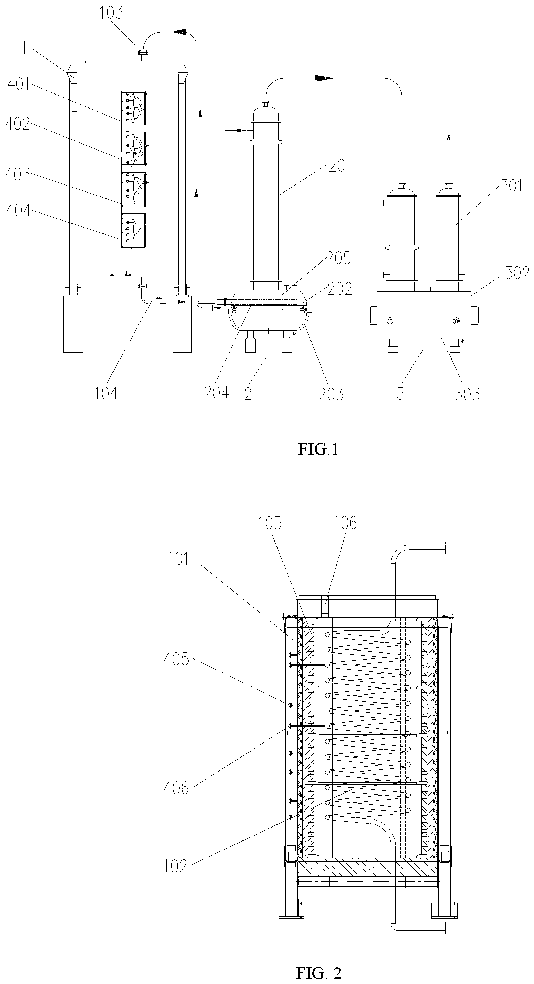

With reference to FIGS. 1 and 2, this embodiment provides a novel anti-coking VDF cracking furnace system, including a cracking furnace 1, a waste heat recoverer 2, and a quencher 3. Firstly, a material is introduced into the cracking furnace 1 and heated for a cracking reaction in the cracking furnace 1, and then a reaction product obtained by the reaction is discharged from the cracking furnace 1 into the waste heat recoverer 2 to perform heat exchange in the waste heat recoverer 2 and preheat the material by using the waste heat of the reaction product, and thus, the material can reach a given temperature before entering the cracking furnace 1, thereby reducing the energy consumption of the cracking furnace; then, the cooled reaction product enters the quencher 3 and is further cooled with a circulating cooling water as cooling medium in the quencher 3 and then discharged to a collection procedure.

Specifically, the above cracking furnace 1 includes a cracking furnace body 101. A spiral furnace tube 102 spiraling down axially along the cracking furnace body is disposed in the cracking furnace body 101. The spiral furnace tube 102 is fixed by a furnace tube holder. A cracking furnace feed opening 103 and a cracking furnace discharge opening 104 are disposed respectively at the top and bottom of the spiral furnace tube 102. The furnace tube is designed as spiral furnace tube structure such that the material is enabled to flow insides at a changing flow rate and be in a continuous descending state, thus avoiding coking due to too high temperature resulting from deposition of the material at a local part of the furnace tube, and further, there is almost no laminar flow of tube wall, the material is uniformly heated and side reaction is reduced. In addition, the above spiral furnace tube 102 spirals down such that all of the furnace tube is close to the inner wall of the cracking furnace body 101. Further, in order to ensure the inner side of the spiral furnace tube 102 is also subjected to heat radiation to allow it to have uniform temperature at inner and outer sides, a spacing of adjacent spiral furnace tubes 102 is set to be three times a diameter of the spiral furnace tube. In this way, the radiation efficiency for the spiral furnace tube is guaranteed and the energy consumption of the cracking furnace is reduced.

The above cracking furnace body is a closed furnace body, in which nitrogen is filled to maintain a micro-positive pressure. An explosion opening 106 is disposed at a side of the cracking furnace body. The explosion opening is connected to an external waste gas treatment system through a pipe line. An explosion disk is disposed on the explosion opening. If the spiral furnace tube explodes, the explosion disk will explode, and a poisonous gas will enter the waste gas treatment system from the explosion opening through a pipe line, avoiding leakage of the poisonous gas.

An up-down zoning heating device 4 is further disposed in the above cracking furnace body. The zoning heating device 4 is disposed at an inner wall of the cracking furnace body 101. The above zoning heating device 4 includes four electric heating zones arranged up and down: a first electric heating zone 401, a second electric heating zone 402, a third electric heating zone 403 and a fourth electric heating zone 404. Each electric heating zone is heated by using an independent electric heating strip. A furnace lining 105 is disposed in the cracking furnace body, and the electric heating strip is disposed on an inner surface of the furnace lining 105.

Each of the above electric heating zones is provided with a furnace wall temperature measuring point 405 and a material temperature measuring point 406. The furnace wall temperature measuring point is disposed on a surface of the electric heating strip, and the material temperature measuring point is disposed at an outer wall of the spiral furnace tube. Each of the furnace wall temperature measuring point and the material temperature measuring point is provided with a thermocouple connected with a temperature control system. Further, the electric heating strip of each electric heating zone is also connected with the temperature control system. The zoning heating device 4 is disposed and the furnace tube is designed as spiral. Further, the zoning heating device and the spiral furnace tube can cooperate with each other, the heating voltage of each electric heating strip is 380/220V and the temperature control system adopts an MPID automatic temperature control system. In this case, the temperature can be controlled to ±1°C. Therefore, the temperature of each electric heating zone can be accurately controlled to keep the material reaction temperature of each zone constant in a case of continuous change of flow rate of the material. In this way, the conversion rate of the cracking reaction is increased, coking resulting from a too high temperature of a zone is avoided, and the energy consumption of the cracking furnace can be reduced.

The above waste heat recoverer 2 includes a first shell-and-tube heat exchanger 201 and a first horizontal tank 202 located at the bottom of the first shell-and-tube heat exchanger 201. A first jacket 203 is disposed at the outside of the first horizontal tank 202. The cracking furnace discharge opening 104 is in communication with a feed opening of the first horizontal tank 202 of the waste heat recoverer 2 through a pipe line, and a discharge opening of the first horizontal tank 202 is in communication with a tube pass feed opening of the first shell-and-tube heat exchanger 201. A cold material is introduced into a shell pass feed opening of the first shell-and-tube heat exchanger 201. A shell pass discharge opening of the first shell-and-tube heat exchanger 201 is in communication with a feed opening of the first jacket 203, and a discharge opening of the first jacket 203 is in communication with the cracking furnace feed opening 103. Further, the shell pass feed opening and the shell pass discharge opening of the first shell-and-tube heat exchanger 201 are located respectively at the top and bottom of the first shell-and-tube heat exchanger 201; the tube pass feed opening and the tube pass discharge opening of the first shell-and-tube heat exchanger 201 are located respectively at the bottom and top of the first shell-and-tube heat exchanger 201.

The above quencher 3 includes two paralleled second shell-and-tube heat exchangers 301 and a second horizontal tank 302 located at the bottom of the two second shell-and-tube heat exchangers 301. A second jacket 303 is disposed at the outside of the second horizontal tank 302. The tube pass discharge opening of the first shell-and-tube heat exchanger 201 is in communication with a top tube pass feed opening of one second shell-and-tube heat exchanger 301 of the quencher through a pipe line, tube pass bottoms of the two second shell-and-tube heat exchangers 301 are both in direct communication with the second horizontal tank 301, and a top tube pass discharge opening of the other second shell-and-tube heat exchanger 301 of the quencher is connected to a product collection procedure through a pipe line. Cooling water is introduced into shell passes of the two second shell-and-tube heat exchangers 301 and the second jacket 301.

Furthermore, in order to ensure the heat exchange rate of the first horizontal tank 202 and interception for a small amount of carbon powder generated by reaction, an inner protruding tube 204 is disposed in the first horizontal tank 202 of the waste heat recoverer. The cracking furnace discharge opening 104 is connected to the inner protruding tube 204 through a pipe line. Further, a vertically-mounted flow guide plate 205 is disposed at a middle upper part of the first horizontal tank 202. The flow guide plate 205 is disposed at a side close to an outlet end of the inner protruding tube. A high temperature reaction product discharged from the inner protruding tube 204, under the action of the flow guide plate 205, firstly flows through the bottom of the first horizontal tank 202 for heat exchange. At the same time, carbon powder in the reaction product is settled down under the action of gravity and the cooling effect of the cold material in the first jacket, so that most of the carbon powder is settled down in the first horizontal tank 202.

The gas flow direction and the heat exchange process in the above cracking furnace system are described below.

The high temperature reaction product from the cracking furnace discharge opening 104 firstly enters the first horizontal tank 202 to perform a first heat exchange with the cold material in the first jacket 203 to preheat the cold material in the first jacket 203 while carbon powder in the reaction product is precipitated in the first horizontal tank 202; then, the reaction product enters the tube pass of the first shell-and-tube heat exchanger 201 from the top discharge opening of the first horizontal tank 202 to perform a second heat exchange with the cold material in the shell pass of the first shell-and-tube heat exchanger 201 to preheat the cold material in the shell pass of the first shell-and-tube heat exchanger 201 and thus the cold material entering from the shell pass of the first shell-and-tube heat exchanger 201 is preheated two times and then conveyed from the discharge opening of the first jacket 203 to the cracking furnace feed opening 103; then, the reaction product enters the tube pass of one second shell-and-tube heat exchanger 301 of the quencher 3 from the tube pass of the first shell-and-tube heat exchanger 201 to perform a first heat exchange with cooling water in the shell pass of the second shell-and-tube heat exchanger and then enters the second horizontal tank 302 at the bottom of the second shell-and-tube heat exchanger to perform a second heat exchange with the cooling water in the second jacket 303 in the second horizontal tank 302 while the second horizontal tank 302 intercepts the carbon powder in the reaction product, and then enters the tube pass of the other second shell-and-tube heat exchanger of the quencher for a third heat exchange with the cooling water in the shell pass and then is discharged from the tube pass discharge opening of the second shell-and-tube heat exchanger to the product collection procedure.

Furthermore, the cold material in the shell pass of the first shell-and-tube heat exchanger 201 enters from top and is discharged into the bottom first horizontal tank whereas the reaction product in the tube pass of the first shell-and-tube heat exchanger 201 enters from bottom and is discharge from top. In this case, countercurrent exchange occurs in the first shell-and-tube heat exchanger 201, thereby increasing the heat exchange efficiency.

This embodiment further provides a use method of the novel anti-coking VDF cracking furnace system. Specifically, the cracking furnace system is applied to prepare vinylidene fluoride through high temperature cracking with 1-chloro-1,1-difluoroethane as raw material, where the reaction temperature is about 750°C. The use method includes the following steps:
(1) the furnace wall temperature measuring point and the material temperature measuring point of each electric heating zone are connected to the temperature control system in advance, and the electric heating strip of the each electric heating zone is connected to the temperature control system, and then a heating temperature range (750±10°C) of each electric heating strip and a temperature range (750±2°C) of the furnace wall temperature measuring point and a temperature range (750±1°C) of the material temperature measuring point of each electric heating zone are set, wherein, when the temperature control system detects the temperatures of the furnace wall temperature measuring point and the material temperature measuring point are not within the corresponding set ranges, the temperature control system automatically adjusts a heating power of the electric heating strip, so as to keep a material reaction temperature constant in the cracking furnace body, that is, keep the temperature of the material temperature measuring point at 750±1°C;
(2) after completing setup according to step (1), a cold material is introduced into the shell pass of the first shell-and-tube heat exchanger of the waste heat recoverer, and passed into the first jacket through the shell pass discharge opening of the first shell-and-tube heat exchanger and then introduced into the cracking furnace feed opening through the discharge opening of the first jacket, then heated to 750°C for reaction in the cracking furnace body, and the temperature control system controls the temperature constant; then a reaction product enters the first horizontal tank through the inner protruding tube from the cracking furnace discharge opening to perform heat exchange with the cold material in the first jacket to preheat the cold material in the first jacket while carbon powder in the reaction product is precipitated in the first horizontal tank; then, the reaction product enters the tube pass of the first shell-and-tube heat exchanger from the top discharge opening of the first horizontal tank to perform heat exchange with the cold material in the shell pass of the first shell-and-tube heat exchanger to preheat the cold material in the shell pass of the first shell-and-tube heat exchanger and the cold material entering from the shell pass of the first shell-and-tube heat exchanger is preheated two times and then conveyed to the cracking furnace feed opening from the discharge opening of the first jacket, where the cold material can be heated to 300°C from normal temperature and the reaction product can be cooled to 300°C;
(3) the reaction product subjected to heat exchange of the waste heat recoverer enters the tube pass of one second shell-and-tube heat exchanger of the quencher to perform a first heat exchange and then enters the second horizontal tank at the bottom of the second shell-and-tube heat exchanger to perform a second heat exchange in the second horizontal tank while the second horizontal tank intercepts the carbon powder in the reaction product, and then enters the tube pass of the other second shell-and-tube heat exchanger of the quencher for a third heat exchange and then is discharged from the top tube pass discharge opening of the second shell-and-tube heat exchanger to the product collection procedure, where, after being subjected to three heat exchanges by the quencher, the reaction product can be cooled to 50 to 60°C.

It is to be noted that those parts not mentioned in the present invention can be achieved by referring to the prior art.

## Claims

1. An anti-coking vinylidene fluoride (VDF) cracking furnace system, comprising a cracking furnace (1), a waste heat recoverer (2) and quencher (3), wherein the cracking furnace comprises a cracking furnace body, a spiral furnace tube (102) and an up-down zoning heating device (4) are disposed in the cracking furnace body, and a cracking furnace feed opening (103) and a cracking furnace discharge opening (104) are disposed respectively at the top and bottom of the spiral furnace tube;
the waste heat recoverer comprises a first shell-and-tube heat exchanger (102) and a first horizontal tank (202) located at the bottom of the first shell-and-tube heat exchanger, a first jacket (203) is disposed on the outside of the first horizontal tank, the cracking furnace discharge opening is in communication with a feed opening of the first horizontal tank of the waste heat recoverer through a pipe line, a discharge opening of the first horizontal tank is in communication with a tube pass feed opening of the first shell-and-tube heat exchanger, the anti-coking VDF cracking furnace system being configured for a cold material to be introduced into a shell pass of the first shell-and-tube heat exchanger, a shell pass discharge opening of the first shell-and-tube heat exchanger is in communication with a feed opening of the first jacket, and a discharge opening of the first jacket is in communication with the cracking furnace feed opening;
the quencher comprises two paralleled second shell-and-tube heat exchangers (301) and a second horizontal tank (302) is disposed at the bottom of the two second shell-and-tube heat exchangers, a tube pass discharge opening of the first shell-and-tube heat exchanger is in communication with a tube pass feed opening of one second shell-and-tube heat exchanger of the quencher through a pipe line, tube pass bottoms of the two second shell-and-tube heat exchangers are both in direct communication with the second horizontal tank, a tube pass discharge opening of the other second shell-and-tube heat exchanger of the quencher is connected through a pipe line to a product collection procedure, and the anti-coking VDF cracking furnace system being configured for cooling water to be introduced into shell passes of the two second shell-and-tube heat exchangers;
the zoning heating device comprises at least two electric heating zones (401; 402) arranged up and down, each electric heating zone is provided with an independent electric heating system, the electric heating system is disposed at an inner wall of the cracking furnace body, each electric heating zone is provided with a furnace wall temperature measuring point (405) and a material temperature measuring point (406), the furnace wall temperature measuring point is disposed at the inner wall of the cracking furnace body, the material temperature measuring point is disposed at an outer wall of the spiral furnace tube, and each of the furnace wall temperature measuring point, the material temperature measuring point and the electric heating system is connected with a temperature control system;
the spiral furnace tube spirals down axially along the cracking furnace body and is close to the inner wall of the cracking furnace body, and a spacing of adjacent spiral furnace tubes is 2 to 4 times a diameter of the spiral furnace tube.

2. The anti-coking VDF cracking furnace system of claim 1, wherein an inner protruding tube is disposed in the first horizontal tank of the waste heat recoverer, and the cracking furnace discharge opening is connected with the inner protruding tube through a pipe line.

3. The anti-coking VDF cracking furnace system of claim 2, wherein a flow guide plate is disposed at a middle upper part of the first horizontal tank, and the flow guide plate is disposed at a side close to an outlet end of the inner protruding tube.

4. The anti-coking VDF cracking furnace system of claim 1, wherein a second jacket is disposed on the outside of the second horizontal tank and cooling water is introduced into the second jacket.

5. A use method of the anti-coking VDF cracking furnace system of any one of claims 1 to 4, comprising the following steps that:
(1) the furnace wall temperature measuring point and the material temperature measuring point of each electric heating zone are connected to the temperature control system in advance, and the electric heating system of the each electric heating zone is connected to the temperature control system, and then a heating temperature range of each electric heating zone and temperature ranges of the furnace wall temperature measuring point and the material temperature measuring point of each electric heating zone are set, wherein, when the temperature control system detects the temperatures of the furnace wall temperature measuring point and the material temperature measuring point are not within the corresponding set ranges, the temperature control system sends signals to automatically adjust a heating power of the electric heating system, so as to keep a material reaction temperature constant in the cracking furnace body;
(2) after completing setup according to step (1), a cold material is introduced into the shell pass of the first shell-and-tube heat exchanger of the waste heat recoverer, and passed into the first jacket through the shell pass discharge opening of the first shell-and-tube heat exchanger and then introduced into the cracking furnace feed opening through the discharge opening of the first jacket to perform reaction in the cracking furnace body, and then a reaction product enters the first horizontal tank and the tube pass of the first shell-and-tube heat exchanger sequentially from the cracking furnace discharge opening so as to perform heat exchange for temperature reduction while the cold material is heated for heat absorption;
(3) the reaction product flows out of the tube pass of the first shell-and-tube heat exchanger and enters the tube pass of one second shell-and-tube heat exchanger, the second horizontal tank and the tube pass of the other second shell-and-tube heat exchanger of the quencher sequentially to perform heat exchange for temperature reduction, and then is discharged from the top tube pass discharge opening of the second shell-and-tube heat exchanger to the product collection procedure.

6. The use method of the anti-coking VDF cracking furnace system of claim 5, wherein, the heat exchange process in step (2) is as follows: the reaction product enters the first horizontal tank through the inner protruding tube from the cracking furnace discharge opening to perform heat exchange with the cold material in the first jacket so as to preheat the cold material of the first jacket; and then, the reaction product enters the tube pass of the first shell-and-tube heat exchanger through the top discharge opening of the first horizontal tank to perform heat exchange with the cold material in the shell pass of the first shell-and-tube heat exchanger so as to preheat the cold material in the shell pass of the first shell-and-tube heat exchanger, that is, the cold material entering from the shell pass of the first shell-and-tube heat exchanger is preheated two times and then conveyed to the cracking furnace feed opening through the discharge opening of the first jacket.

7. The use method of the anti-coking VDF cracking furnace system of claim 5, wherein, the heat exchange process in step (3) is as follows: the reaction product enters the tube pass of one second shell-and-tube heat exchanger of the quencher from the tube pass of the first shell-and-tube heat exchanger to perform a first heat exchange with the cooling water in the shell pass of the second shell-and-tube heat exchanger and then enters the second horizontal tank at the bottom of the second shell-and-tube heat exchanger to perform a second heat exchange with the cooling water in the second jacket in the second horizontal tank, and then enters the tube pass of the other second shell-and-tube heat exchanger of the quencher for a third heat exchange and then is discharged from the tube pass discharge opening of the second shell-and-tube heat exchanger to the product collection procedure.

## Patentansprüche

1. Ein Antiverkokung-Vinylidenfluorid (VDF)-Spaltofensystem, umfassend einen Spaltofen (1), eine Abwärmerückgewinnungseinheit (2) und einen Quencher (3),
wobei der Spaltofen einen Spaltofenkörper umfasst, ein Spiralofenrohr (102) und eine Auf/Abwärts-Zonenheizvorrichtung (4) in dem Spaltofenkörper angeordnet sind, und eine Spaltofenbeschickungsöffnung (103) und eine Spaltofenausgabeöffnung (104) jeweils an dem oberen und unteren Ende des Spiralofenrohrs angeordnet sind;
die Abwärmerückgewinnungseinheit einen ersten Rohrbündelwärmetauscher (102) und einen ersten horizontalen Tank (202), der sich am Boden des ersten Rohrbündelwärmetauschers befindet, umfasst, wobei ein erster Mantel (203) an der Außenseite des ersten horizontalen Tanks angeordnet ist, die Spaltofenausgabeöffnung über eine Rohrleitung mit einer Beschickungsöffnung des ersten horizontalen Behälters der Abwärmerückgewinnungseinheit in Verbindung ist, eine Ausgabeöffnung des ersten horizontalen Tanks mit einer RohrdurchgangBeschickungsöffnung des ersten Rohrbündelwärmetauschers in Verbindung ist, das Antiverkokung-VDF-Spaltofensystem konfiguriert ist, damit ein kaltes Material in einen Manteldurchgang des ersten Rohrbündelwärmetauschers eingeführt wird, eine Manteldurchgangausgabeöffnung des ersten Rohrbündelwärmetauschers mit einer Beschickungsöffnung des ersten Mantels in Verbindung ist, und eine Ausgabeöffnung des ersten Mantels mit der Spaltofenbeschickungsöffnung in Verbindung ist;
der Quencher zwei parallele zweite Rohrbündelwärmetauscher (301) umfasst und ein zweiter horizontaler Tank (302) an dem Boden der zwei zweiten Rohrbündelwärmetauscher angeordnet ist, eine Rohrdurchgangausgabeöffnung des ersten Rohrbündelwärmetauschers mit einer Rohrdurchgangbeschickungsöffnung eines zweiten Rohrbündelwärmetauschers des Quenchers über eine Rohrleitung in Verbindung ist, die Rohrdurchgangböden der zwei zweiten Rohrbündelwärmetauscher beide in direkter Verbindung mit dem zweiten horizontalen Behälter sind, eine Rohrdurchgangausgabeöffnung des anderen zweiten Rohrbündelwärmetauschers des Quenchers über eine Rohrleitung mit einem Produktsammelverfahren verbunden ist, und das Antiverkokung-VDF-Spaltofensystem konfiguriert ist, um Kühlwasser in die Manteldurchgänge der zwei zweiten Rohrbündelwärmetauscher einzuleiten;
die Zonenheizvorrichtung mindestens zwei elektrische Heizzonen (401; 402) umfasst, die oben und unten angeordnet sind, jede elektrische Heizzone mit einem unabhängigen elektrischen Heizsystem versehen ist, das elektrische Heizsystem an einer Innenwand des Spaltofenkörpers angeordnet ist, jede elektrische Heizzone mit einem Ofenwandtemperaturmesspunkt (405) und einem Materialtemperaturmesspunkt (406) versehen ist,
der Ofenwandtemperaturmesspunkt an der Innenwand des Spaltofenkörpers angeordnet ist, der Materialtemperaturmesspunkt an einer Außenwand des Spiralofenrohrs angeordnet ist, und jeder von dem Ofenwandtemperaturmesspunkt, dem Materialtemperaturmesspunkt und dem elektrischen Heizsystem mit einem Temperaturregelsystem verbunden ist;
das Spiralofenrohr sich spiralförmig axial entlang des Spaltofenkörpers nach unten windet und nahe an der Innenwand des Spaltofenkörpers ist, und der Abstand von benachbarten Spiralofenrohren das 2- bis 4-fache des Durchmessers des Spiralofenrohrs ist.

2. Antiverkokung-VDF-Spaltofensystem nach Anspruch 1, wobei ein inneres hervorstehendes Rohr in dem ersten horizontalen Tank der Abwärmerückgewinnungseinheit angeordnet ist, und die Spaltofenausgabeöffnung durch eine Rohrleitung mit dem inneren hervorstehenden Rohr verbunden ist.

3. Antiverkokung-VDF-Spaltofensystem nach Anspruch 2, wobei eine Strömungsleitplatte an einem mittleren oberen Teil des ersten horizontalen Tanks angeordnet ist, und die Strömungsleitplatte an einer Seite nahe dem Auslassende des inneren hervorstehenden Rohrs angeordnet ist.

4. Antiverkokung-VDF-Spaltofensystem nach Anspruch 1, wobei ein zweiter Mantel an der Außenseite des zweiten horizontalen Tanks angeordnet ist und Kühlwasser in den zweiten Mantel eingeleitet wird.

5. Verwendungsverfahren des Antiverkokung-VDF-Spaltofensystems nach einem der Ansprüche 1 bis 4, umfassend die folgenden Schritte:
(1) der Ofenwandtemperaturmesspunkt und der Materialtemperatur jeder elektrischen Heizzone sind im Voraus mit dem Temperaturregelsystem verbunden, und das elektrische Heizsystem jeder elektrischen Heizzone ist mit dem Temperaturregelsystem verbunden, und dann werden ein Heiztemperaturbereich von jeder elektrischen Heizzone und Temperaturbereiche des Ofenwandtemperaturmesspunkts und des Materialtemperaturmesspunkts von jeder elektrischen Heizzone eingestellt, wobei, wenn das Temperaturregelsystem erkennt, dass die Temperaturen des Ofenwandtemperaturmesspunkts und des Materialtemperaturmesspunkts nicht innerhalb der entsprechenden eingestellten Bereiche sind, das Temperaturregelsystem Signale sendet, um automatisch eine Heizleistung des elektrischen Heizsystems einzustellen, um eine Materialreaktionstemperatur in dem Spaltofenkörper konstant zu halten;
(2) nach Abschluss des Einstellens gemäß Schritt (1) wird ein kaltes Material in den Manteldurchgang des ersten Rohrbündelwärmetauschers der Abwärmerückgewinnungseinheit eingeleitet und durch die Manteldurchgangausgabeöffnung des ersten Rohrbündelwärmetauschers in den ersten Mantel geleitet, und dann durch die Ausgabeöffnung des ersten Mantels in die Spaltofenbeschickungsöffnung eingeleitet, um die Reaktion in dem Spaltofenkörper durchzuführen, und dann tritt ein Reaktionsprodukt von der Spaltofenausgabeöffnung aus nacheinander in den ersten horizontalen Behälter und den Rohrdurchgang des ersten Rohrbündelwärmetauschers ein, um einen Wärmeaustausch zur Temperatursenkung durchzuführen, während das kalte Material zur Wärmeaufnahme erhitzt wird;
(3) das Reaktionsprodukt aus dem Rohrdurchgang des ersten Rohrbündelwärmetauschers fließt und nacheinander in den Rohrdurchgang eines zweiten Rohrbündelwärmetauschers, in den zweiten horizontalen Tank und in den Rohrdurchgang des anderen zweiten Rohrbündelwärmetauschers des Quenchers eintritt, um einen Wärmeaustausch zur Temperatursenkung durchzuführen, und dann wird es aus der oberen Rohrdurchgangausgabeöffnung des zweiten Rohrbündelwärmetauschers in das Produktsammelverfahren ausgegeben.

6. Verwendungsverfahren des Antiverkokung-VDF-Spaltofensystems nach Anspruch 5, wobei
der Wärmeaustauschprozess in Schritt (2) wie folgt ist: das Reaktionsprodukt tritt durch das innere hervorstehende Rohr von der Spaltofenausgabeöffnung in den ersten horizontalen Tank ein, um einen Wärmeaustausch mit dem kalten Material in dem ersten Mantel durchzuführen, um das kalte Material des ersten Mantels vorzuheizen, und dann tritt das Reaktionsprodukt durch die obere Ausgabeöffnung des ersten horizontalen Tanks in den Rohrdurchgang des ersten Rohrbündelwärmetauschers ein, um einen Wärmeaustausch mit dem kalten Material in dem Mantelgang des ersten Rohrbündelwärmetauschers durchzuführen, um das kalte Material in dem Manteldurchgang des ersten Rohrbündelwärmetauschers vorzuheizen, das heißt, das kalte Material, das aus dem Manteldurchgang des ersten Rohrbündelwärmetauschers eintritt, wird zweimal vorgeheizt und dann durch die Ausgabeöffnung des ersten Mantels zu der Spaltofenbeschickungsöffnung befördert.

7. Verwendungsverfahren des Antiverkokung-VDF-Spaltofensystems nach Anspruch 5, wobei
der Wärmeaustauschprozess in Schritt (3) wie folgt ist: das Reaktionsprodukt tritt in den Rohrdurchgang eines zweiten Rohrbündelwärmetauschers des Quenchers aus dem Rohrdurchgang des ersten Rohrbündelwärmetauschers ein, um einen ersten Wärmeaustausch mit dem Kühlwasser in dem Rohrdurchgang des zweiten Rohrbündelwärmetauschers durchzuführen und dann tritt es in den zweiten horizontalen Tank an dem Boden des zweiten Rohrbündelwärmetauschers ein, um einen zweiten Wärmeaustausch mit dem Kühlwasser in dem zweiten Mantel des zweiten horizontalen Tanks durchzuführen, und dann tritt es für einen dritten Wärmeaustausch in den Rohrdurchgang des anderen zweiten Rohrbündelwärmetauschers des Quenchers ein und wird dann aus der Rohrdurchgangausgabeöffnung des zweiten Rohrbündelwärmetauschers in das Produktsammelverfahren ausgegeben.

## Revendications

1. Système de four de craquage de fluorure de vinylidène (VDF) anti-cokéfaction, comprenant un four de craquage (1), un récupérateur de chaleur perdue (2) et un extincteur (3), dans lequel le four de craquage comprend un corps de four de craquage, un tube de four hélicoïdal (102) et un dispositif de chauffage par zones haut-bas (4) qui sont disposés dans le corps de four de craquage, et un orifice d'alimentation de four de craquage (103) et un orifice d'évacuation de four de craquage (104) sont disposés respectivement au niveau du haut et du bas du tube de four hélicoïdal ;
le récupérateur de chaleur perdue comprend un premier échangeur de chaleur à calandre (102) et un premier réservoir horizontal (202) situé en bas du premier échangeur de chaleur à calandre, une première chemise (203) est disposée sur l'extérieur du premier réservoir horizontal, l'orifice d'évacuation du four de craquage est en communication avec un orifice d'alimentation du premier réservoir horizontal du récupérateur de chaleur perdue à travers une canalisation, un orifice d'évacuation du premier réservoir horizontal est en communication avec un orifice d'alimentation du passage de tube du premier échangeur de chaleur à calandre, le système de four de craquage de VDF anti-cokéfaction étant configuré pour qu'un matériau froid soit introduit dans un passage de calandre du premier échangeur de chaleur à calandre, un orifice d'évacuation de passage de calandre du premier échangeur de chaleur à calandre est en communication avec un orifice d'alimentation de la première chemise, et un orifice d'évacuation de la première chemise est en communication avec l'orifice d'alimentation de four de craquage ;
l'extincteur comprend deux deuxièmes échangeurs de chaleur à calandre mis en parallèle (301) et un deuxième réservoir horizontal (302) est disposé en bas des deux deuxièmes échangeurs de chaleur à calandre, un orifice d'évacuation de passage de tube du premier échangeur de chaleur à calandre est en communication avec un orifice d'alimentation de passage de tube d'un deuxième échangeur de chaleur à calandre de l'extincteur à travers une canalisation, les bas de passage de tube des deux deuxièmes échangeurs de chaleur à calandre sont tous deux en communication directe avec le deuxième réservoir horizontal, un orifice d'évacuation de passage de tube de l'autre deuxième échangeur de chaleur à calandre de l'extincteur est relié par le biais d'une canalisation à une procédure de collecte de produit, et le système de four de craquage de VDF anti-cokéfaction étant configuré pour refroidir l'eau devant être introduite dans des passages de calandre des deux deuxièmes échangeurs de chaleur à calandre ;
le dispositif de chauffage par zones comprend au moins deux zones de chauffage électrique (401 ; 402) agencées en haut et en bas, chaque zone de chauffage électrique est pourvue d'un système de chauffage électrique indépendant, le système de chauffage électrique est disposé au niveau d'une paroi interne du corps de four de craquage, chaque zone de chauffage électrique est pourvue d'un point de mesure de température de paroi de four (405) et d'un point de mesure de température de matériau (406), le point de mesure de température de paroi de four est disposé au niveau d'une paroi interne du corps de four de craquage, le point de mesure de température de matériau est disposé au niveau d'une paroi externe du tube de four hélicoïdal, et chacun du point de mesure de température de paroi de four, du point de mesure de température de matériau et du système de chauffage électrique est relié à un système de régulation de température ;
le tube de four hélicoïdal descend en spirale axialement le long du corps de four de craquage et est proche de la paroi interne du corps de four de craquage, et un espacement des tubes de four hélicoïdaux adjacents est égal à 2 à 4 fois un diamètre du tube de four hélicoïdal.

2. Système de four de craquage de VDF anti-cokéfaction selon la revendication 1, dans lequel un tube en saillie interne est disposé dans le premier réservoir horizontal du récupérateur de chaleur perdue, et l'orifice d'évacuation du four de craquage est relié au tube en saillie interne par le biais d'une canalisation.

3. Système de four de craquage de VDF anti-cokéfaction selon la revendication 2, dans lequel une plaque guide de flux est disposée au niveau d'une partie supérieure centrale du premier réservoir horizontal, et la plaque guide de flux est disposée au niveau d'un côté proche d'une extrémité de sortie du tube en saillie interne.

4. Système de four de craquage de VDF anti-cokéfaction selon la revendication 1, dans lequel une deuxième chemise est disposée sur l'extérieur du deuxième réservoir horizontal, et de l'eau de refroidissement est introduite dans la deuxième chemise.

5. Procédé d'utilisation du système de four de craquage de VDF anti-cokéfaction selon l'une quelconque des revendications 1 à 4, comprenant les étapes suivantes où :
1) le point de mesure de température de la paroi de four et le point de mesure de la température du matériau de chaque zone de chauffage électrique sont reliés au système de régulation de température à l'avance, et le système de chauffage électrique de chaque zone de chauffage électrique est relié au système de régulation de température, puis une plage de températures de chauffage de chaque zone de chauffage électrique et des plages de température du point de mesure de température de paroi du four et du point de mesure de température de matériau de chaque zone de chauffage électrique sont définies, dans lequel, lorsque le système de régulation de température détecte que les températures du point de mesure de température de paroi du four et du point de mesure de température de matériau ne sont pas dans les plages définies correspondantes, le système de régulation de température envoie des signaux pour ajuster automatiquement une puissance de chauffage du système de chauffage électrique, de sorte à maintenir une température de réaction de matériau constante dans le corps de four de craquage ;
(2) après l'achèvement de l'installation selon l'étape (1), un matériau froid est introduit dans le passage de calandre du premier échangeur de chaleur à calandre du récupérateur de chaleur perdue, et passé dans la première chemise à travers l'orifice d'évacuation de passage de calandre du premier échangeur de chaleur à calandre et introduit ensuite dans l'orifice d'alimentation de four de craquage à travers l'orifice d'évacuation de la première chemise pour réaliser une réaction dans le corps de four de craquage, puis un produit de réaction pénètre dans le premier réservoir horizontal et dans le passage de tube du premier échangeur de chaleur à calandre séquentiellement à partir de l'orifice d'évacuation de four de craquage de sorte à réaliser un échange de chaleur pour une réduction de température tandis que le matériau froid est chauffé pour une absorption de chaleur ;
(3) le produit de réaction s'écoule hors du passage de tube du premier échangeur de chaleur à calandre et pénètre dans le passage de tube d'un deuxième échangeur de chaleur à calandre, du deuxième réservoir horizontal et du passage de tube de l'autre deuxième échangeur de chaleur à calandre de l'extincteur séquentiellement pour réaliser un échange de chaleur pour une réduction de température, puis est évacué par l'orifice d'évacuation de passage de tube haut du deuxième échangeur de chaleur à calandre vers la procédure de collecte de produit.

6. Procédé d'utilisation du système de four de craquage de VDF anti-cokéfaction selon la revendication 5, dans lequel, le processus d'échange de chaleur de l'étape (2) est le suivant : le produit de réaction pénètre dans le premier réservoir horizontal à travers le tube en saillie interne à partir de l'orifice d'évacuation de four de craquage pour réaliser un échange de chaleur avec le matériau froid dans la première chemise de sorte à préchauffer le matériau froid de la première chemise ; puis, le produit de réaction pénètre dans le passage de tube du premier échangeur de chaleur à calandre à travers l'orifice d'évacuation haut du premier réservoir horizontal pour réaliser un échange de chaleur avec le matériau froid dans le passage de calandre du premier échangeur de chaleur à calandre de sorte à préchauffer le matériau froid dans le passage de calandre du premier échangeur de chaleur à calandre, c'est-à-dire que le matériau froid pénétrant depuis le passage de calandre du premier échangeur de chaleur à calandre est préchauffé deux fois puis acheminé vers l'orifice d'alimentation de four de craquage à travers l'orifice d'évacuation de la première chemise.

7. Procédé d'utilisation du système de four de craquage de VDF anti-cokéfaction selon la revendication 5, dans lequel, le processus d'échange de chaleur de l'étape (3) est le suivant : le produit de réaction pénètre dans le passage de tube d'un deuxième échangeur de chaleur à calandre de l'extincteur depuis le passage de tube du premier échangeur de chaleur à calandre pour réaliser un premier échange de chaleur avec l'eau de refroidissement dans le passage de calandre du deuxième échangeur de chaleur à calandre puis pénètre dans le deuxième réservoir horizontal au niveau du bas du deuxième échangeur de chaleur à calandre pour réaliser un deuxième échange de chaleur avec l'eau de refroidissement dans la deuxième chemise dans le deuxième réservoir horizontal, puis pénètre dans le passage de tube de l'autre deuxième échangeur de chaleur à calandre de l'extincteur pour un troisième échange de chaleur et est ensuite évacué par l'orifice d'évacuation de passage de tube du deuxième échangeur de chaleur à calandre vers la procédure de collecte de produit.
